# EUROPÄISCHE PATENTSCHRIFT

(11) **EP 0 836 845 B1**
(45) Veröffentlichungstag und Bekanntmachung des Hinweises auf die Patenterteilung: **26.05.2004**
(21) Anmeldenummer: 97250222.3
(22) Anmeldetag: 29.07.1997
(51) Int. Cl.: A61K 6/083

(54) **Dentaler lichthärtender Opaker**
Light curable opaque dental material
Matériau dentaire opaque durcissable par la lumière

(30) Priorität: 26.08.1996 DE 19634460; 03.09.1996 DE 19635667
(43) Veröffentlichungstag der Anmeldung: 22.04.1998
(73) Patentinhaber: Ivoclar Vivadent AG, 9494 Schaan (LI)
(72) Erfinder: Voser, Dieter, 9494 Schaan (LI); Zanghellini, Gerhard, 9494 Schaan (LI); Rheinberger, Volker, Dr., 9490 Vaduz (LI)
(74) Vertreter: UEXKÜLL & STOLBERG

(56) Entgegenhaltungen:
- EP-A- 0 011 190
- DE-A- 3 332 179
- US-A- 2 533 196
- US-A- 4 500 658

## Beschreibung

Die Erfindung betrifft einen dentalen lichthärtenden Opaker, der sich insbesondere durch eine hohe Deckkraft und dennoch gute Polymerisierbarkeit bis in tiefe Schichten auszeichnet und sich daher vorteilhaft zum Abdecken von metallischen Oberflächen von Dentalrestaurationen eignet.

Bei der Verblendung von metallischen Zahnkronen oder -brücken werden sogenannte Opaker eingesetzt, welche auch als Grundiermassen bezeichnet werden. Die Opaker dienen zur Abdeckung des Metallgerüstes der Dentalrestaurationen, damit ein Durchscheinen des dunklen Metalles verhindert und somit mit der anschließend aufgebrachten relativ dünnen Verblendung die Farbe der natürlichen Zähne simuliert werden kann. Neben einer hohen Deckkraft muß der Opaker auch eine gute Haftung sowohl an dem Metallgerüst als auch an der Verblendung aufweisen, damit die endgültige Zahnrestauration in sich stabil ist.

Dentale Opaker sind aus dem Stand der Technik bekannt.

So beschreibt die DE-C-33 32 179 photopolymerisierbare dentale Opaker, die Photopolymerisationskatalysator und eine Mischung aus Zirkoniumdioxid und Titandioxid als Pigment enthalten. Der Opaker wird als dünne Schicht auf ein Metallgerüst aufgetragen und anschließend mit Licht ausgehärtet. Das mit dem Opaker bedeckte Metallgerüst wird dann mit Kunststoff verblendet und zum Beispiel zu einer Zahnkrone ausgehärtet. Dabei kommt es jedoch nicht zu einer zufriedenstellenden Durchhärtung des Opakers.

Aus der DE-C-41 19 483 sind pastenförmige Grundiermassen für Keramik- und Kunststoffverblendungen bekannt, die bei hoher Temperatur eingebrannt werden und Glaspulver sowie als Trübungsmittel Zirkoniumdioxid enthalten. Zur Einfärbung der Grundiermassen werden diesen gegebenenfalls Farbpigmente zugemischt.

Weiter ist es aus den Stand der Technik bekannt, Perlpolymerisate als Komponente von Zahnfüllmassen oder von Materialien zur Herstellung von Prothesen einzusetzen.

So beschreibt EP-B-11 186 Perlpolymerisate von viskosen Dimethacrylaten, welche als Füllmittel in pastenförmigen Dentalmassen eingesetzt werden können.

EP-B-11 735 und EP-B-11 734 beschreiben pastenförmige röntgenopake Zahnfüllmassen, welche mit Füllstoff versehene Perlpolymerisate enthalten. Als Füllstoffe werden dabei zum Beispiel Bariumsulfat und Siliciumdioxid eingesetzt. Farbpigmente sind jedoch in dem Perlpolymerisat nicht vorhanden.

Weiter beschreibt EP-B-84 769 vernetzte Perlpolymerisate, die insbesondere Siliciumdioxid als Füllstoff, jedoch wiederum keine Farbpigmente enthalten. Die Perlpolymerisate werden als Komponente von Zahnfüllmaterialien eingesetzt, wobei sie sich zum Abdecken von unerwünscht gefärbten Materialien, wie Metallen, nicht eignen, da sie eine hohe Transparenz aufweisen.

Mit Füllstoffen gefüllte vernetzte Perlpolymerisate und deren Einsatz als Füllmittel in pastenförmigen Dentalmassen sind ebenfalls aus der EP-B-11 190 bekannt. Als Füllstoffe kommen insbesondere Magnesiumhydroxidcarbonat, Titandioxid, Bariumsulfat, Zirkoniumdioxid und Siliciumdioxid in Frage. Sowohl die Einarbeitung von Farbpigment in die Perlpolymerisate als auch die Verwendung der Perlpolymerisate als Komponente von dentalen Opakern werden jedoch nicht beschrieben.

Schließlich offenbart US-A-4,500,658 röntgenopake Acrylperlpolymerisate, welche zur Herstellung von Dentalprothesen eingesetzt werden können. Die Röntgenopazität der Polymerisate wird durch eingearbeitetes röntgenopakes Pigment hervorgerufen. Bei der Verarbeitung der Polymerisate zu Dentalprothesen erfolgt Härtung durch Wärme und nicht durch Bestrahlung mit Licht. Weiter wird es ebenfalls nicht offenbart, die röntgenopaken Polymerisate als dentale Opaker zum Abdecken von Metallgerüsten bei der Herstellung von Kronen oder Brücken einzusetzen.

Bei bekannten Opakern, die durch Licht gehärtet werden, tritt das besondere Problem auf, daß diese infolge des hohen Gehaltes von abdeckenden Pigmenten, wie z.B. TiO₂ und ZrO₂ nur in unbefriedigendem Maße bei Bestrahlung mit Licht aushärten. Dies ist eine Schwierigkeit, die naturgemäß bei wärmehärtenden Materialien nicht auftritt. Eine Reduzierung des Gehaltes an abdeckenden Pigmenten ist nicht möglich, da diese für eine gute Deckkraft erforderlich sind. Somit sind zwei Anforderungen zu erfüllen, nämlich hohe Deckkraft und vollständige Härtung durch Licht, die einander zuwiderlaufen, da die Pigmente das Eindringen des Lichtes in tiefere Schichten des aufgebrachten Opakers und damit eine vollständige Polymerisation verhindern.

So ist es bei konventionellen lichthärtenden Pastenopakern bekannt, daß diese häufig nur an der obersten Schicht aushärten und darunter weich und unpolymerisiert sind.

Ein weiteres bei konventionellen Pastenopakern auftretendes Problem ist, daß sie bei längerer Lagerung dazu neigen, sich zu entmischen, wodurch eine füllstoffreiche und eine füllstoffarme Phase entsteht.

Der Erfindung liegt demgemäß die Aufgabe zugrunde, einen dentalen lichthärtenden Opaker zur Verfügung zu stellen, der trotz einer hohen Deckkraft durch Licht auch in tiefergelegenen Schichten ausgehärtet werden kann und welcher auch bei längerer Lagerung keine Entmischung zeigt.

Diese Aufgabe wird durch den dentalen lichthärtenden Opaker nach den Ansprüchen 1 bis 12 gelöst.

Die Erfindung betrifft weiter die Verwendung des Opakers nach Anspruch 13.

Der erfindungsgemäße dentale lichthärtende Opaker ist dadurch gekennzeichnet, daß er folgende Komponenten enthält:
(a) vernetztes Perlpolymerisat, in das ein farbiges Pigment eingearbeitet ist, und
(b) polymerisierbares polyfunktionelles Monomer,
wobei die eingesetzte Menge an (a) in der eingesetzten Menge an (b) nicht vollständig löslich ist.

Dabei sind die eingesetzten Perlpolymerisate vorzugsweise auf der Basis von mindestens einem (Meth)acrylsäureester.

Vorteilhafte Perlpolymerisate (a) enthalten 1 bis 40, insbesondere 5 bis 20 und besonders bevorzugt 10 bis 15 Gew.-% eingearbeitetes farbiges Pigment, bezogen auf die Menge an Perlpolymerisat.

Weiter hat es sich als vorteilhaft erwiesen, daß das Perlpolymerisat einen Vernetzungsgrad von 10 bis 90, insbesondere 20 bis 80 und besonders bevorzugt 40 bis 60 % aufweist.

Typischerweise liegt das Perlpolymerisat in Form von Teilchen mit einer mittleren Teilchengröße von 10 bis 100 und insbesondere 12 bis 30 µm, bezogen auf die Anzahl der Teilchen, vor.

Als farbiges Pigment können übliche zur Einfärbung von Dentalwerkstoffen geeignete Farbpigmente verwendet werden. Besonders bevorzugt sind dabei Ultramarinblau; Eisenoxid-Pigmente; farbige Pigmente auf Basis von Kobalt-, Aluminium-, Chrom-, Nickel- und/oder Zink-Oxiden; und/oder organische farbige Pigmente. Besonders bevorzugt sind dabei rote und gelbe Eisenoxid-Pigmente. Schwarze und weiße Pigmente, wie ZrO₂ oder TiO₂, sind erfindungsgemäß nicht als farbige Pigmente geeignet, weshalb diese nicht unter dem Begriff "farbige Pigmente" im Sinne der Erfindung fallen.

Zur Herstellung des in dem Opaker eingesetzten vernetzten Perlpolymerisates werden bekannte Verfahren verwendet. Diese sind beispielsweise in den oben bereits erwähnten Patenten EP-B-11 190, EP-B-84 769, EP-B-11 735, EP-B-11 734 und US-A-4,500,658 und der darin jeweils zitierten Literatur beschrieben. Wichtig ist dabei, daß die Polymerisation in Gegenwart des farbigen Pigmentes erfolgt, so daß dieses nach Abschluß der Polymerisation in den entstehenden Perlen eingearbeitet ist.

Weiter enthält der erfindungsgemäße lichthärtende Opaker polymerisierbares polyfunktionelles Monomer (b). Hierfür kommen bei Dentalmaterialien eingesetzte polyfunktionelle (Meth)acrylate in Frage. Besonders vorteilhaft sind dabei Ethylenglycoldimethacrylat (EGDMA), Triethylenglycoldimethacrylat (TEGDMA), Hexandioldimethacrylat, 2,2-Bis-4-(3-methacryloxy-2-hydroxy-propoxy)-phenylpropan (Bis-GMA), durch Umsetzung von 2,2,4-Trimethylhexamethylendiisocyanat mit 2-Hydroxyethylmethacrylat entstehende Urethandimethacrylate, Decandioldimethacrylat (D₃MA), Butandioldimethacrylat sowie die entsprechenden Acrylate.

Die eingesetzte Menge an mit farbigem Pigment versehenem vernetzten Perlpolymerisat darf in der verwendeten Menge an Monomer nicht vollständig löslich sein. Ein Anlösen des Perlpolymerisats in dem polyfunktionellen Monomer (b) und gegebenenfalls vorhandenem monofunktionellen Monomer (f) ist jedoch möglich. Weiter ist es unerwünscht, daß eine Quellung des Perlpolymerisats in den eingesetzten Monomeren stattfindet, da sich ansonsten die Viskosität des Opakers zu sehr ändern würde. Ein Anstieg der Viskosität würde dabei zum Beispiel bedeuten, daß der Opaker nur sehr schlecht aus den üblicherweise eingesetzten Applizierspritzen ausgedrückt werden könnte.

Damit der erfindungsgemäße Opaker in einem breiten Bereich eine farbliche Anpassung an natürliches Zahnmaterial gestattet, enthält er neben dem farbigen Pigment üblicherweise auch weißes Pigment (c1) und/oder schwarzes Pigment (c2). Dabei werden bevorzugt als weißes Pigment ZrO₂ und/oder TiO₂ und als schwarzes Pigment Ruß-Pigment eingesetzt. ZrO₂ ist besonders bevorzugt, da es selbst in sehr hohen Gehalten die Durchhärtung nicht behindert.

Weiter enthält der erfindungsgemäße Opaker üblicherweise einen Photoinitiator (d), der die Photopolymerisation katalysiert. Zu diesem Zweck können insbesondere Benzophenon und seine Derivate sowie Benzoin und seine Derivate eingesetzt werden. Weitere bevorzugte Photoinitiatoren sind die α-Diketone, wie 9,10-Phenanthrenchinon, Diacetyl, Furil, Anisil, 4,4'-Dichlorbenzyl und 4,4'-Dialkoxybenzyl. Besonders bevorzugt wird Campherchinon eingesetzt. Außerdem ist es in vielen Fällen vorteilhaft, die Photoinitiatoren zusammen mit Reduktionsmitteln einzusetzen, wofür insbesondere Amine, wie Cyanethylmethylanilin, Dimethylaminoethylmethacrylat, Triethylamin, Triethanolamin, N,N-Dimethylanilin, n-Methyldiphenylamin, N,N-Dimethyl-sym.-xyliden, N,N-3,5-Tetramethylanilin und 4-Dimethylaminobenzoesäureethylester, in Frage kommen.

Gegebenenfalls können in dem Opaker auch anorganische Füllstoffe enthalten sein. Für diesen Zweck finden vornehmlich gefällte oder pyrogene Kieselsäuren mit einer mittleren Primärteilchengröße von 5 bis 100 nm und einer BET-Oberfläche von 0 bis 400 m²/g Anwendung. Derartige Materialien werden unter der Bezeichnung

Aerosil® von der Firma Degussa oder HDK® von der Firma Wacker vertrieben.

Schließlich kann der erfindungsgemäße Opaker auch noch polymerisierbare monofunktionelle (Meth)acrylsäureester, insbesondere Methylmethacrylat (MMA), Hydroxyethylmethacrylat, Tetrahydrofurfurylmethacrylat, Cyclohexylmethacrylat, Isobutylmethacrylat sowie die entsprechenden Acrylate enthalten.

Der erfindungsgemäße Opaker wird in üblicher Weise, insbesondere durch inniges Vermischen der eingesetzten Komponenten, hergestellt.

Hierbei kann insbesondere so vorgegangen werden, daß zunächst vernetztes Perlpolymerisat mit eingearbeitetem farbigen Pigment, zum Beispiel rote und gelbe vernetzte Perlpolymerisate, hergestellt werden. Zu diesem Zwecke wird eine Vormischung von Vernetzern mit roten oder gelben Farbpigmenten mit Methylmethacrylat gemischt, und zu dieser Mischung wird Peroxid als Polymerisationsstarter zugegeben. Als Vernetzer finden typischerweise polyfunktionelle Methacrylsäureester, wie zum Beispiel Urethandimethacrylate und Triethylenglycoldimethacrylat, Verwendung. Die Polymerisation wird dann in bekannter Weise in wäßriger Phase als Suspensionspolymerisation durchgeführt. Die erhaltenen roten oder gelben Perlpolymerisate werden abgetrennt, getrocknet und gesiebt. Üblicherweise schließt sich dann noch ein Temperungsschritt an, um eventuell vorhandenes Restperoxid zu zerstören.

Mit den so hergestellten roten und gelben Perlpolymerisate werden dann Farbpasten hergestellt. Dazu werden polymerisierbares polyfunktionelles Monomer (b) sowie gegebenenfalls weitere Komponenten, wie Photoinitiator (d) und anorganischer Füllstoff (e), mit den Perlpolymerisaten gemischt. Die Mischung wird dann üblicherweise auf einem Dreiwalzenstuhl homogenisiert, wodurch lichthärtbare rote und gelbe Farbpasten entstehen. Zur Anpassung der Farbe dieser Pasten an die gewünschte Farbe eines natürlichen Zahnes werden sie mit konventionellen weißen und/oder schwarzen Farbpasten gemischt. Diese konventionellen weißen und schwarzen Farbpasten werden dadurch erhalten, daß polymerisierbares polyfunktionelles Monomer, Photoinitiator und anorganischer Füllstoff mit schwarzen Pigmenten oder mit weißen Pigmenten, insbesondere ZrO₂, homogenisiert wird.

Der erfindungsgemäße lichthärtende Opaker eignet sich in vorteilhafter Weise zum Abdecken von Metallgerüsten in der Dentaltechnik. Dazu wird er insbesondere auf die Metallgerüste von dentalen Brücken oder Kronen aufgebracht und gehärtet, und anschließend wird auf das mit dem Opaker versehene Metallgerüst eine Verblendung, insbesondere eine Kunststoffverblendung, in üblicher Weise aufgebracht.

Dabei hat es sich überraschenderweise gezeigt, daß der erfindungsgemäße Opaker auch bei hohem Gehalt an Pigmenten, insbesondere bei als weißem Pigment verwendetem ZrO₂, und einer damit einhergehenden hohen Deckkraft, dennoch im wesentlichen vollständig mittels Licht gehärtet werden kann. Konventionelle lichthärtende Opaker zeigen hingegen nur eine geringe Durchhärtungstiefe.

Weiter neigt der erfindungsgemäße Opaker auch nicht zur Entmischung unter Bildung einer füllstoffreichen und einer füllstoffarmen Phase, weshalb er sich auch für eine längere Lagerung eignet.

Schließlich zeigt der erfindungsgemäße Opaker auch eine sehr gute Haftung sowohl an unterschiedlichen Metallen, die dentale Gerüststrukturen bilden können, als auch an aufgebrachten Verblendungen.

Besonders vorteilhafte Mengen der einzelnen Komponenten des erfindungsgemäßen Opakers sind nachstehend angegeben, wobei diese unabhängig voneinander gewählt werden können:
(a) 2 bis 20, insbesondere 2 bis 15 und besonders bevorzugt 3 bis 12 Gew.-% Perlpolymerisat mit eingearbeitetem farbigen Pigment,
(b) 20 bis 70, insbesondere 40 bis 60 und besonders bevorzugt 50 bis 52 Gew.-% polymerisierbares polyfunktionelles Monomer,
(c1) 20 bis 60, insbesondere 25 bis 50 und besonders bevorzugt 30 bis 45 Gew.-% weißes Pigment,
(c2) 0 bis 0,1, insbesondere 0 bis 0,01 und besonders bevorzugt 0 bis 0,005 Gew.-% schwarzes Pigment,
(d) 0,1 bis 6, insbesondere 0,5 bis 4,5 und besonders bevorzugt 1 bis 3 Gew.-% Photoinitiator,
(e) 0 bis 20, insbesondere 2 bis 20 und besonders bevorzugt 5 bis 6 Gew.-% anorganischer Füllstoff,
   und/oder
(f) 1 bis 6, insbesondere 1,5 bis 4,5 und besonders bevorzugt 2 bis 3 Gew.-% polymerisierbarer monofunktioneller (Meth)acrylsäureester.

Die Erfindung wird im folgenden anhand von Beispielen näher erläutert.

### Beispiele

### Beispiel 1

### Herstellung von rotem und gelbem vernetzten Perlpolymer

Zunächst wurde eine Grundmischung nachstehender Zusammensetzung hergestellt:

| Grundmischung | |
|---|---|
| Urethandimethacrylat | 3,0 g |
| TEGDMA | 0,530 g |
| Butylkresol | 0,710 g |
| Kieselsäure (HDK H-15) | 0,350 g |
| Methylmethacrylat | 4,100 g |

Diese Grundmischung wurde jeweils getrennt mit 1,1 g eines roten Eisenoxid-Farbpigmentes (Siccotrans rot) bzw. 1,1 g eines gelben Eisenoxid-Farbpigmentes (Siccotrans gelb) vermischt. Mit diesen Mischungen wurden in einem Reaktor nach den Methoden der Suspensionspolymerisation, daß heißt unter Rühren und Hitze, rote und gelbe Perlpolymerisate hergestellt. Die Korngröße konnte dabei durch die Polymerisationsparameter eingestellt werden, und sie lag bei den hergestellten Perlpolymeren unterhalb von 32 µm.

### Beispiel 2

### Farbpasten

Die in Beispiel 1 hergestellten roten und gelben Perlpolymerisate wurde zur Herstellung von erfindungsgemäßen roten und gelben Opaker-Farbpasten verwendet. Gleichfalls wurden konventionelle weiße und schwarze Farbpasten hergestellt. In jedem Fall wurden die einzelnen Komponenten unter Rühren gemischt und zu einer Paste in einem Dreiwalzenstuhl homogenisiert. Die einzelnen roten, gelben, weißen und schwarzen Farbpasten hatten die folgende Zusammensetzung.

| | rot (Gew.-%) | gelb (Gew.-%) | weiß (Gew.-%) | schwarz (Gew.-%) |
|---|---|---|---|---|
| BisGMA | 44,6 | 44,6 | 42,9 | - |
| TEGDMA | 5 | 5 | 5 | - |
| HDK-2000 | 3,5 | 3,5 | 5 | - |
| Perlpolymer | 40,0 | 40,0 | - | - |
| ZrO₂ | - | - | 40,0 | - |
| Campherchinon | 0,7 | 0,7 | 0,9 | - |
| EMBO | 1,0 | 1,0 | 1,0 | - |
| Decandioldimethacrylat | 5,2 | 5,2 | 5,2 | - |
| Ruß-Pigment (Microlith) | - | - | - | 30 |
| Kolophoniumharz | - | - | - | 70 |

Dabei ist die Bedeutung von verwendeten Abkürzungen wie folgt:
- HDK-15: Mikrofeines hydrophiles SiO₂, BET-Oberfläche 150 m²/g
- HDK-2000: Mikrofeines hydrophobes SiO₂, BET-Oberfläche 170 m²/g
- EMBO: p-Dimethylaminobenzoesäureethylester

### Beispiel 3 - Herstellung von Opakern

Es wurden verschiedenfarbige pastenförmige Opaker unter Verwendung der in Beispiel 2 beschriebenen Farbpasten hergestellt.

Dabei wurden die Anteile der einzelnen Farbpasten in den nachstehend angegebenen Bereichen variiert, um eine breite farbliche Anpaßbarkeit zu erzielen.

| | |
|---|---|
| Farbpaste gelb | 6,4 bis 24,0 Gew.-% |
| Farbpaste rot | 0,1 bis 10,0 Gew.-% |
| Farbpaste weiß | 74,0 bis 93,0 Gew.-% |
| Farbpaste schwarz | 0,5 bis 4,5 Gew.-%. |

Zur Herstellung des fertigen Opakers wurden die einzelnen Farbpasten zusammengewogen und in einem Rührwerk hochtourig homogenisiert. Die erhaltenen Opaker hatten eine Durchhärtungstiefe, die deutlich größer war als die von vergleichbaren Opakern nach dem Stand der Technik.

Zum Vergleich mit konventionellen Opakern wurde ein rötlicher erfindungsgemäßer Opaker hergestellt, dessen Farbe der Farbbezeichnung 540 des Chromascopfarbringes der Firma Ivoclar, Liechtenstein, nahe kommt. Hierzu wurden die in Beispiel 2 beschriebenen Farbpasten weiß, rot, gelb und schwarz gemischt.

Zum Vergleich wurde ein gleichfarbiger konventioneller Opaker hergestellt, indem man Farbpasten weiß, rot, gelb und schwarz zu einer entsprechenden Farbe zusammenmischte. Dabei enthielten die Farbpasten rot und gelb jedoch keine farbigen Perlpolymerisate, sondern es wurden lediglich die entsprechenden Farbpigmente, nämlich Eisenoxid rot und Eisenoxid gelb, zugegeben.

Man pinselte dann von beiden Opakern eine dünne Schicht auf Metall auf, polymerisierte diese Schicht in einem Lichthärtungs-Gerät, nämlich Spectramat der Firma Ivoclar, Liechtenstein, für 2 Minuten, trug eine zweite Schicht deckend auf und polymerisierte für weitere 5 Minuten in dem Lichthärtungsgerät. Dann wurde die Opakerschicht mit einer Sonde abgekratzt.

Bei dem erfindungsgemäßen Opaker war die untere Schicht durchgehend hart und auspolymerisiert, während der konventionelle Opaker ohne Gehalt an Perlpolymerisat mit eingearbeitetem Farbpigment eine weiche und unpolymerisierte untere Schicht aufwies.

## Patentansprüche

1. Dentaler lichthärtender Opaker, **dadurch gekennzeichnet, daß** er folgende Komponenten enthält:
(a) vernetztes Perlpolymerisat, in das ein farbiges Pigment eingearbeitet ist, und
(b) polymerisierbares polyfunktionelles Monomer,
wobei die eingesetzte Menge an (a) in der eingesetzten Menge an (b) nicht vollständig löslich ist.

2. Opaker nach Anspruch 1, **dadurch gekennzeichnet, daß** das Perlpolymerisat (a) auf der Basis von mindestens einem (Meth)acrylsäurester ist.

3. Opaker nach Anspruch 1 oder 2, **dadurch gekennzeichnet, daß** das Perlpolymerisat (a) 1 bis 40, insbesondere 5 bis 20 und besonders bevorzugt 10 bis 15 Gew.-% eingearbeitetes farbiges Farbpigment enthält, bezogen auf Masse Perlpolymerisat.

4. Opaker nach einem der Ansprüche 1 bis 3, **dadurch gekennzeichnet, daß** das Perlpolymerisat einen Vernetzungsgrad von 10 bis 90, insbesondere 20 bis 80 und besonders bevorzugt 40 bis 60 % aufweist.

5. Opaker nach einem der Ansprüche 1 bis 4, **dadurch gekennzeichnet, daß** das Perlpolymerisat eine mittlere Teilchengröße von 10 bis 100 und insbesondere 12 bis 30 µm, bezogen auf die Anzahl der Teilchen, hat.

6. Opaker nach einem der Ansprüche 1 bis 5, **dadurch gekennzeichnet, daß** er als farbiges Pigment Ultramarinblau; Eisenoxid-Pigmente; farbige Pigmente auf Basis von Kobalt-, Aluminium-, Chrom-, Nickel- und/oder Zink-Oxiden; und/oder organische farbige Pigmente enthält.

7. Opaker nach einem der Ansprüche 1 bis 6, **dadurch gekennzeichnet, daß** er als weitere Komponente
(c1) weißes Pigment und/oder
(c2) schwarzes Pigment
enthält.

8. Opaker nach Anspruch 7, **dadurch gekennzeichnet, daß** er
- ZrO₂ und/oder TiO₂ als weißes Pigment und/oder
- Ruß-Pigment als schwarzes Pigment
enthält.

9. Opaker nach einem der Ansprüche 1 bis 8, **dadurch gekennzeichnet, daß** er als weitere Komponente
(d) Photoinitiator
enthält.

10. Opaker nach einem der Ansprüche 1 bis 9, **dadurch gekennzeichnet, daß** er als weitere Komponente
(e) anorganischen Füllstoff
enthält.

11. Opaker nach einem der Ansprüche 1 bis 10, **dadurch gekennzeichnet, daß** er als weitere Komponente
(f) polymerisierbaren monofunktionellen (Meth)acrylsäureester
enthält.

12. Opaker nach einem der Ansprüche 1 bis 11, **dadurch gekennzeichnet, daß** die Mengen der Komponenten unabhängig voneinander wie folgt sind:
(a) 2 bis 20 Gew.-% Perlpolymerisat mit eingearbeitetem farbigen Pigment,
(b) 20 bis 70 Gew.-% polymerisierbares polyfunktionelles Monomer,
(c1) 20 bis 60 Gew.-% weißes Pigment,
(c2) 0 bis 0,1 Gew.-% schwarzes Pigment,
(d) 0,1 bis 6 Gew.-% Photoinitiator,
(e) 0 bis 20 Gew.-% anorganischer Füllstoff,
und/oder
(f) 1 bis 6 Gew.-% polymerisierbarer monofunktioneller (Meth)acrylsäureester.

13. Verwendung des Opakers gemäß einem der Ansprüche 1 bis 12 zum Abdecken von Metallgerüsten einer dentalen Brücke- oder Krone.

## Claims

1. Dental light-curable opaquer **characterised in that** it comprises the following components:
(a) a cross-linked bead polymer into which a coloured pigment is incorporated, and
(b) a polymerisable polyfunctional monomer,
with the proviso that the quantity of (a) used is not completely soluble in the quantity of (b) used.

2. Opaquer according to Claim 1 **characterised in that** the bead polymer (a) is based on at least one (meth)acrylic acid ester.

3. Opaquer according to Claim I or 2 **characterised in that** the bead polymer (a) comprises 1 to 40, in particular 5 to 20 and particularly preferably 10 to 15 wt.% of incorporated coloured pigment, relative to the quantity of bead polymer.

4. Opaquer according to any one of Claims 1 to 3 **characterised in that** the bead polymer has a degree of cross-linking of 10 to 90, in particular 20 to 80 and particularly preferably 40 to 60%.

5. Opaquer according to any one of Claims 1 to 4 **characterised in that** the bead polymer has a number-average particle size of 10 to 100 and in particular 12 to 30µ, based on the number of particles.

6. Opaquer according to any one of Claims 1 to 5 **characterised in that** it comprises as the coloured pigment ultramarine blue; iron oxide pigments; coloured pigments based on cobalt, aluminium, chromium, nickel and/or zinc oxides; and/or organic coloured pigments.

7. Opaquer according to any one of Claims 1 to 6 **characterised in that** it comprises as a further component
(c1) white pigment and/or
(c2) black pigment.

8. Opaquer according to Claim 7 **characterised in that** it comprises
- ZrO₂ and/or TiO₂ as white pigment and/or
- carbon black pigment as black pigment.

9. Opaquer according to any one of Claims 1 to 8 **characterised in that** it comprises as a further component
(d) photoinitiator.

10. Opaquer according to any one of Claims 1 to 9 **characterised in that** it comprises as a further component
(e) inorganic filler.

11. Opaquer according to any one of Claims 1 to 10 **characterised in that** it comprises as a further component
(f) polymerisable monofunctional (meth)acrylic acid ester.

12. Opaquer according to any one of Claims 1 to 11 **characterised in that** the quantities of the components independently of one another are as follows:
(a) 2 to 20 wt.% of bead polymer with incorporated coloured pigment,
(b) 20 to 70 wt.% of polymerisable polyfunctional monomer,
(c1) 20 to 60 wt.% of white pigment,
(c2) 0 to 0.1 wt.% of black pigment,
(d) 0.1 to 6 wt.% of photoinitiator,
(e) 0 to 20 wt.% of inorganic filler,
and/or
(f) 1 to 6 wt.% of polymerisable monofunctional (meth)acrylic acid esters.

13. Use of the opaquer according to any one of Claims 1 to 12 for covering metal structures of a dental bridge or crown.

## Revendications

1. Matériau dentaire opaque durcissant à la lumière, **caractérisé en ce qu'**il contient les composants suivants :
(a) un polymère perlé réticulé dans lequel un pigment coloré est incorporé, et
(b) un monomère polyfonctionnel polymérisable,
où la quantité utilisée de (a) n'est pas entièrement soluble dans la quantité utilisée de (b).

2. Matériau dentaire opaque selon la revendication 1, **caractérisé en ce que** le polymère perlé utilisé (a) est de préférence à base d'au moins un ester d'acide (méth)acrylique.

3. Matériau dentaire opaque selon la revendication 1 ou 2, **caractérisé en ce que** le polymère perlé (a) contient de 1 à 40, en particulier de 5 à 20 et particulièrement préférentiellement de 10 à 15 % en poids de pigment coloré incorporé par rapport à la masse de polymère perlé.

4. Matériau dentaire opaque selon l'une des revendications 1 à 3, **caractérisé en ce que** le polymère perlé présente un degré de réticulation de 10 à 90, en particulier de 20 à 80 et particulièrement préférentiellement de 40 à 60 %.

5. Matériau dentaire opaque selon l'une des revendications 1 à 4, **caractérisé en ce que** le polymère perlé se présente sous forme de particules ayant une taille moyenne de particules de 10 à 100 et en particulier de 12 à 30 µm, par rapport au nombre de particules.

6. Matériau dentaire opaque selon l'une des revendications 1 à 5, **caractérisé en ce qu'**il contient comme pigment coloré du bleu outremer ; des pigments d'oxyde de fer ; des pigments colorés à base d'oxydes de cobalt, d'aluminium, de chrome, de nickel et/ou de zinc ; et/ou des pigments colorés organiques.

7. Matériau dentaire opaque selon l'une des revendications 1 à 6, **caractérisé en ce qu'**il contient comme autres composants
(c1) un pigment blanc et/ou
(c2) un pigment noir.

8. Matériau dentaire opaque selon la revendication 7, **caractérisé en ce qu'**il contient
- du ZrO₂ et/ou du TiO₂ comme pigment blanc et/ou
- du pigment de suie comme pigment noir.

9. Matériau dentaire opaque selon l'une des revendications 1 à 8, **caractérisé en ce qu'**il comprend comme autres composants
(d) un photoinitiateur.

10. Matériau dentaire opaque selon l'une des revendications 1 à 9, **caractérisé en ce qu'**il comprend comme autres composants
(e) une charge inorganique.

11. Matériau dentaire opaque selon l'une des revendications 1 à 10, **caractérisé en ce qu'**il comprend comme autre composant
(f) de l'ester d'acide (méth)acrylique monofonctionnel polymérisable.

12. Matériau dentaire opaque selon l'une des revendications 1 à 11, **caractérisé en ce que** les quantités des composants sont, indépendamment les unes des autres, les suivantes :
(a) 2 à 20 % en poids de polymère perlé avec un pigment coloré incorporé,
(b) 20 à 70 % en poids de monomère polyfonctionnel polymérisable,
(c1) 20 à 60 % en poids de pigment blanc,
(c2) 0 à 0,1 % en poids de pigment noir,
(d) 0,1 à 6 % en poids de photoinitiateur,
(e) 0 à 20 % en poids de charge inorganique,
et/ou
(f) 1 à 6 % en poids d'ester d'acide (méth)acrylique monofonctionnel polymérisable.

13. Utilisation du matériau dentaire opaque selon l'une quelconque des revendications 1 à 12 pour couvrir les squelettes métalliques d'une couronne ou d'un bridge dentaire.
